# EUROPEAN PATENT APPLICATION

(11) **EP 4 491 627 A1**
(43) Date of publication of application: **15.01.2025**
(21) Application number: 23382728.6
(22) Date of filing: 14.07.2023
(51) Int. Cl.: C07D 491/052, A61P 25/02, A61P 25/04, A61P 25/16, A61P 25/28, A61P 29/00, A61K 31/519

(54) **SYNTHETIC CANNABINOIDS BASED ON CHROMENOPYRIMIDINE SCAFFOLD AND USE THEREOF**

(71) Applicant: Consejo Superior De Investigaciones Científicas, 28006 Madrid (ES); Universitat de Barcelona, 08028 Barcelona (ES)
(72) Inventor: Jagerovic, Nadine, Madrid (ES); Morales, Paula, Madrid (ES); Figuerola Asencio, Laura, Madrid (ES); Franco Fernández, Rafael, Barcelona (ES); Navarro Brugal, Gemma, Barcelona (ES)
(74) Representative: Pons

(57) **Abstract**

The present invention relates to biologically active compounds of formula (I) capable of interacting with the CB1 and/or the CB2 cannabinoid receptors. The present invention also relates to the use of said compounds in the treatment and/or prevention of CB1 and/or CB2 cannabinoid receptors related diseases.

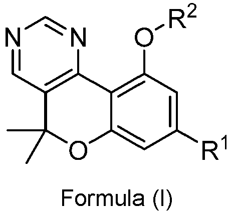

## Description

The present invention relates to biologically active compounds, based on chromenopyrimidine scaffold, capable of interacting with the CB1 and/or the CB2 cannabinoid receptors. The present invention also relates to the use of said compounds in the treatment and/or prevention of CB1 and/or CB2 cannabinoid receptors related diseases.

Therefore, the present invention belongs to the field of medicine.

### BACKGROUND ART

Phytocannabinoids have raised interest for the management of diverse disorders. Unfortunately, the abuse potential of phytocannabinoids constitutes a limitation to their therapeutic value. Over the past decade, synthetic cannabinoid receptor agonists have been studied as possible therapeutic agents for a variety of diseases.

The endocannabinoid system has emerged as an important neuromodulatory system. Thus, compounds targeting this system have therapeutic potential for the clinical management of inflammatory disorders, neuropathic pain, and neurological pathologies including neurodegenerative processes such as including Alzheimer's, Parkinson's, and Huntington's diseases. Therefore, exogenous cannabinoids may play an important role in the regulation of neuronal function.

The intrinsic complexity of the endocannabinoid system is currently faced by cannabinoids as medicines. Its therapeutic exploitation is essentially developed for phytocannabinoids. Only few CB1/CB2 synthetic cannabinoids could enter clinical phases. However, their efficacy determined in preclinical assays did not reach the expectation, as anti-inflammatory for instance. Thus, new cannabinoid scaffolds could bring new properties and/or improved pharmacodynamics and pharmacokinetics.

Structurally, the compounds claimed in the present invention are related to previous chromenopyrazoles having cannabinoid properties (WO2010109050) replacing the pyrazole ring by a pyrimidine. This modification lead to the new claimed compounds having cannabinoid functionality shown in primary neuronal cultures.

The synthesis of a compound having a chromenopyrimidine scaffold, 5,5-dimethyl-8-pentyl-5H-chromeno[4,3-d]pyrimidin-10-ol related to Formula (I) has been described in Bellassoud-Fargeau et al. Heterocycles 1986, 24 (10), 2831. The structure of 5,5-dimethyl-8-pentyl-2-phenyl-5H-chromeno[4,3-d]pyrimidin-10-ol has been published in Greb et al. Tetrahedron Letters 1972, 6, 545 and in Bellassoud-Fargeau et al. Heterocycles 1986, 24 (10), 2831. However, no biological data have been reported for these three compounds so far. Therefore, the activity of the compounds claimed in this patent application brings novelty to the field.

The present invention provides a new group of compounds capable of interacting with the CB1 and/or the CB2 cannabinoid receptors useful for treating neurological or inflammatory disorders related to said receptors.

### SUMMARY OF THE INVENTION

The invention relates to pyrimidine derivatives which are modulators of cannabinoid receptor 2 (CB2) and/or cannabinoid receptor 1 (CB1) and which are useful for the treatment or prevention of neurological or inflammatory disorders in which these receptors are involved.

In one aspect, the present invention relates to a compound of formula (I) or a pharmaceutically acceptable salt thereof: 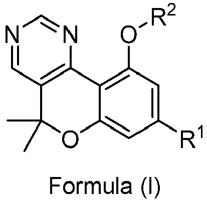 wherein:
R¹ is selected from substituted or unsubstituted C₁-C₉ alkyl;
R² is selected from hydrogen, substituted or unsubstituted C₁-C₆ alkyl, substituted or unsubstituted C₅-C₂₀ aryl, substituted or unsubstituted C₃-C₉ cycloalkyl, or substituted or unsubstituted heterocyclic ring,
for use as a medicament.

In a preferred embodiment of the compounds for use, R¹ is selected from methyl, pentyl, 1,1-cyclopentylheptyl and 1,1-dimethylheptyl and, even more preferably, R¹ is 1,1-dimethylheptyl. The substituent 1,1-cyclopentylheptyl refers to a substituent of formula:

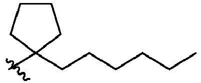

In a preferred embodiment of the compound for use, R² is a substituted or unsubstituted C₁-C₄ alkyl group, more preferably, R² is a C₁-C₄ alkyl group selected from methyl, ethyl, iso-propyl, tert-butyl and, even more preferably, R² is methyl (Me).

In another preferred embodiment of the compound for use, R² is a C₁-C₄ alkyl group substituted by an aryl group, preferably phenyl. Even more preferably, R² is a benzyl group (Bn).

In another preferred embodiment of the compound for use, R² is a substituted or unsubstituted C₅-C₇ cycloalkyl group, more preferably, R² is cyclohexyl.

In another preferred embodiment of the compound for use, R² is a substituted or unsubstituted C₅-C₁₂ aryl group, more preferably R² is selected from phenyl and phenyl substituted by a hydroxyl or a methoxy group.

In another preferred embodiment of the compound for use, R² is H

In a more preferably embodiment, the compound of formula (I) for use is selected from the list comprising:
8-(1,1-dimethylheptyl)-5,5-dimethylchromeno[4,3-d]pyrimidin-10-ol,
8-(1,1-dimethylheptyl)-10-methoxy-5,5-dimethylchromeno[4,3-d]pyrimidine, and
10-benzyloxy-8-(1,1-dimethylheptyl)-5,5-dimethylchromeno[4,3-d]pyrimidine.

Hereinafter, the compounds of formula (I) or their pharmaceutically acceptable salts will be referred to as compounds of the invention.

Another aspect of the present invention relates to a compound of formula (I), as defined above, or a pharmaceutically acceptable salt thereof for use in the prevention and/or the treatment of inflammatory disorders associated with the cannabinoid receptors selected from neuropathic pain and neurodegenerative diseases selected from: Alzheimer's, Parkinson's and Huntington's diseases.

Another aspect of the present invention relates to a compound of formula (I) or a pharmaceutically acceptable salt thereof: 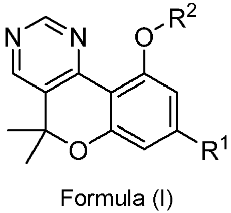 wherein:
R¹ is selected from substituted or unsubstituted C₁-C₉ alkyl;
R² is selected from hydrogen, substituted or unsubstituted C₁-C₆ alkyl, substituted or unsubstituted C₅-C₂₀ aryl, substituted or unsubstituted C₃-C₉ cycloalkyl, or substituted or unsubstituted heterocyclic ring;
and provided that the compound 5,5-dimethyl-8-pentyl-5H-chromeno[4,3-d]pyrimidin-10-ol is excluded from the formula (I).

In a preferred embodiment of the compound of the invention, R¹ is selected from methyl, pentyl, 1,1-cyclopentylheptyl and 1,1-dimethylheptyl and, even more preferably, R¹ is 1,1-dimethylheptyl.

In a preferred embodiment of the compound, R² is a substituted or unsubstituted C₁-C₄ alkyl group, more preferably, R² is a C₁-C₄ alkyl group selected from methyl, ethyl, iso-propyl, tert-butyl and, even more preferably, R² is methyl.

In another preferred embodiment of the compound for use, R² is a C₁-C₄ alkyl group substituted by an aryl group, preferably phenyl. Even more preferably, R² is a benzyl group.

In another preferred embodiment of the compound, R² is a substituted or unsubstituted C₅-C₇ cycloalkyl group, more preferably, R² is cyclohexyl.

In another preferred embodiment of the compound, R² is a substituted or unsubstituted C₅-C₁₂ aryl group, more preferably R² is selected from phenyl and phenyl substituted by a hydroxyl or a methoxy group.

In another preferred embodiment of the compound, R² is H

In a more preferably embodiment, the compound of formula (I) is selected from the list comprising:
8-(1,1-dimethylheptyl)-5,5-dimethylchromeno[4,3-d]pyrimidin-10-ol,
8-(1,1-dimethylheptyl)-10-methoxy-5,5-dimethylchromeno[4,3-d]pyrimidine, and
10-benzyloxy-8-(1,1-dimethylheptyl)-5,5-dimethylchromeno[4,3-d]pyrimidine.

The compounds of formula (I) may be in crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

Unless otherwise indicated, the compounds of the invention also include compounds which differ only in the presence of one or more isotopically enriched atoms. For example, compounds which have said structure, with the exception of the substitution of a hydrogen for a deuterium or for tritium, or the substitution of a carbon for a ¹³C or ¹⁴C enriched carbon or a ¹⁵N enriched nitrogen, they are within the scope of this invention.

Another aspect of the present invention relates to a pharmaceutical composition comprising a compound of formula (I) or a pharmaceutically acceptable salt thereof and at least one excipient, adjuvant and/or pharmaceutically acceptable carrier.

The pharmaceutical compositions can be administered by any suitable administration route, for example: oral, parenteral (subcutaneous, intraperitoneal, intravenous, intramuscular, etc.), rectal, etc.

In a particular embodiment, said pharmaceutical compositions may be in a pharmaceutical form of oral administration, either solid or liquid. Illustrative examples of pharmaceutical forms of oral administration include tablets, capsules, granules, solutions, suspensions, etc., and may contain conventional excipients such as binders, dilutes, disintegrating agents, lubricants, humectants, etc., and may be prepared by conventional methods. The pharmaceutical compositions may also be adapted for parenteral administration, in the form of, for example, solutions, suspensions or lyophilised, sterile products in the suitable dosage form; in this case, said pharmaceutical compositions will include suitable excipients, such as buffers, surfactants, etc. In any case, the excipients are chosen according to the pharmaceutical form of administration selected.

The present invention also relates to a method for the prevention and/or treatment of inflammatory disorders associated with the cannabinoid receptors selected from neuropathic pain, neurodegenerative diseases selected from Alzheimer's, Parkinson's, and Huntington's diseases, said method comprises administering, to a patient in need thereof, a therapeutically effective quantity of a compound of formula (I) as previously defined.

The term "therapeutically effective quantity" means the necessary quantity of a compound for the treatment or prevention of the disease, disorder or condition to be effective. It will also depend on the subject to be treated, the severity of the illness suffered by said subject, the chosen form of administration, etc.

### Definitions

The term "pharmaceutically acceptable salt", as used herein, refers to salts of such compounds that are appropriate for use in pharmaceutical contexts, *i.e.,* salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, the ones approved by a regulatory agency of a federal or state government or collected in the US Pharmacopoeia or other generally recognised pharmacopeia for use in animals and, more particularly, in humans.

The term "excipients, adjuvants and/or carriers" relates to molecular entities or substances through which the active ingredient is administered. Such pharmaceutical excipients, adjuvants or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes.

The term "treatment or prevention" as used herein, unless otherwise indicated, relates to reversing, alleviating and inhibiting the progress of, or preventing the disorder or condition to which it applies in such terms, one or more symptoms of such disorder or condition.

The term "alkyl" refers to a saturated, straight or branched hydrocarbon chain and which bind to the rest of the molecule by a single bond, for example, methyl, ethyl, *n-*propyl, *i*-propyl, *n*-butyl, *terc*-butyl, *sec*-butyl, *n*-pentyl, etc. The number of carbons of the hydrocarbon chain in a specific alkyl group is indicated just before the term "alkyl". Thus, for example, "C₁-C₉ alkyl" refers to an alkyl group that have between 1 and 9 carbon atoms. The alkyl groups may be optionally substituted by one or more substituents, such cycloalkyl (preferably C₁-C₆ cycloalkyl), aryl (preferably C₅-C₇ aryl), halogen (F, Cl, Br or I), hydroxy (-OH), or alkoxy (preferably-O-C₁-C₄ alkyl) substituents.

The term " aryl" refers to a mono-, bi- or poly- carbocyclic aromatic system and which bind to the rest of the molecule by a single bond. The number of carbons of the aryl group is indicated just before the term "aryl". Thus, for example, "C₅-C₂₀ aryl" refers to an aryl group that have between 5 and 20 carbon atoms. Examples of aryl groups are phenyl, biphenyl, naphthyl and anthracyl. The aryl groups of this invention can be optionally substituted by one or more substituents, such as alkyl (preferably C₁-C₅ alkyl), cycloalkyl (preferably C₁-C₆ cycloalkyl), aryl (preferably C₅-C₇ aryl), halogen (F, Cl, Br or I), hydroxy (-OH), or alkoxy (preferably-O-C₁-C₄ alkyl) substituents

The term "cycloalkyl" refers to an optionally substituted saturated ring monocyclic or polycyclic system and which bind to the rest of the molecule by a single bond. The number of carbons in a specific cycloalkyl group is indicated just before the term "cycloalkyl". Thus, for example, "C₃-C₉ cycloalkyl" refers to a cycloalkyl group that have between 3 and 9 carbon atoms. Exemplary monocyclic cycloalkyl rings include cyclopropyl, cyclobutyl, cyclopentyl, cyclohexyl, and cycloheptyl. Cycloalkyl groups may be optionally substituted by one or more substituents, such as alkyl (preferably C₁-C₅ alkyl), aryl (preferably C₅-C₇ aryl), halogen (F, Cl, Br or I), hydroxy (-OH), or alkoxy (preferably-O-C₁-C₄ alkyl) substituents.

The term "heterocyclic ring" refers to a 4-, 5-, 6- or 7-membered ring containing one, two or three heteroatoms independently selected from the group consisting of nitrogen, oxygen and sulfur. Examples are pyrazole, triazole, furane, thiazole. They can be saturated or include doble bonds. heterocyclic ring groups may be optionally substituted by one or more substituents, such as alkyl (preferably C₁-C₅ alkyl), cycloalkyl (preferably C₁-C₆ cycloalkyl), aryl (preferably C₅-C₇ aryl), halogen (F, Cl, Br or I), hydroxy (-OH), or alkoxy (preferably-O-C₁-C₄ alkyl) substituents.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**Fig. 1****.** Graphics of the dose-response corresponding to the HTFR (Homogeneous Time Resolved Fluorescence) assays performed with chromenopyrimidines Example 1, Example 2 and Example 3. A) Competition assay snap-CB1; red ligand used at 100 nM; B) Competition assay snap-CB2; red ligand used at 20 nM.
**Fig. 2****.** cAMP (Cyclic adenosine monophosphate) experiments performed with novel chromenopyrimidines (Example 1, Example 2 and Example 3). A) In HEK293 cells transfected with CB1; B) In HEK293 cells transfected with CB2.
**Fig. 3****.** Graphics of the dose-response corresponding to the MAPK (mitogen-activated protein kinase) experiments performed with novel chromenopyrimidines (Example 1, Example 2 and Example 3). A) In HEK293 cells transfected with CB1; B) In HEK293 cells transfected with CB2.
**Fig. 4****.** Graphics of the dose-response corresponding to the β-arrestin2 recruitment assays performed with Example 1, Example 2 and Example 3. A) In HEK293 cells transfected with CB1; B) In HEK293 cells transfected with CB2.
**Fig. 5****.** Graphics of the dose-response corresponding to the cAMP assays performed with Example 1, Example 2 and Example 3 in neurons.
**Fig. 6****.** Graphics of the dose-response of reference cannabinoids ACEA and anandamide in cAMP and MAPK assays in HEK293-CB1 cells.
**Fig. 7****.** Graphics of the dose-response of reference cannabinoids JWH133 and anandamide in cAMP and MAPK assays in HEK293-CB2 cells.

### Examples

The invention will now be illustrated by assays carried out by the inventors, which shows the effectiveness of the product of the invention.

### General Methods of Preparation

Reagents and solvents were used without further purification or drying. Products were purified using flash column chromatography (Merck Silica gel 60, 230-400 mesh) or medium pressure chromatography using Biotage Isolera One with pre-packed silica gel columns (Biotage SNAP cartridges). The structure of the described compounds was determined by a combination of nuclear magnetic resonance (NMR) and mass spectrum (MS) data. Their purity was controlled by high performance liquid chromatography coupled to mass spectrometry (HPLC-MS) analysis and high-resolution mass spectrometry (HRMS). NMR spectra were recorded on a Varian INNOVA 300 (300 and 75 MHz), Bruker400 (400 and 101 MHz), or a Varian 500 (500 and 126 MHz) at 25 °C and 40 °C. Samples were prepared as solutions in deuterated solvent and referenced to internal non-deuterated solvent peak. Chemical shifts were expressed in ppm (δ) downfield of tetramethylsilane. HPLC-MS analysis was performed on a Waters 2695 HPLC system equipped with a photodiode array 2996 coupled to Micromass ZQ 2000 mass spectrometer (ESI-MS), using a reverse-phase column SunFire^{™} (C-18, 2.1 × 50 mm, 3.5 µm) in a 10 min gradient A: CH₃CN/0.1 % formic acid, B: H₂O/0.1 % formic acid visualizing at λ = 254 nm. Flow rate was 1 mL/min. HRMS experiments were performed in a LC-MS hybrid quadrupole/time of flight (QTOF) analyzer equipped with an Agilent 1100 LC coupled to an Agilent 6500 Accurate Mass (1-2 ppm mass accuracy) using electrospray ionization in the positive mode (ESI⁺).

A process is described in Scheme 1 for the preparation of compounds of the present invention of general formula I.

### Scheme 1. Synthetic pathway for the preparation of Example 1, Example 2, and Example 3:

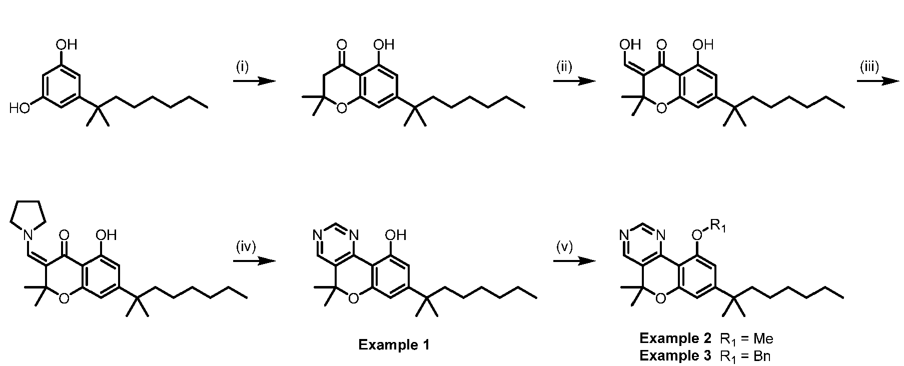

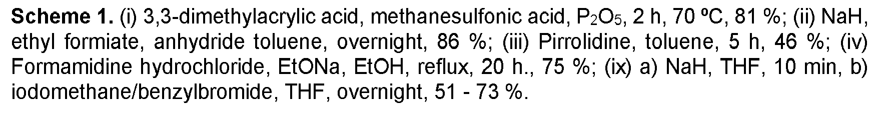

The compounds of general formula (I) of the present invention were synthesized from commercially (Chemosapiens) available 5-alkylresorcinol as illustrated in Scheme 1. 5-Alkylresorcinol was treated with 3,3-dimethylacrylic acid, methanesulfonic acid, and phosphate pentoxide under nitrogen atmosphere. The resulting 7-alkyl-5-hydroxy-2,2-dimethylchroman-4-one was let to react with sodium hydride and ethyl formate to lead to 7-alkyl-5-hydroxy-3-hydrodymethylene-2,2-dimethylchroman-4-one. This later treated with pyrrolidine allowed the preparation of the intermediate 7-alkyl-5-hydroxy-3-(pyrrolidine-1-yl-methylene)-2,2-dimethylchroman-4-one. Finally, the formation of the pyrimidine was done by treatment with a solution of formamidine with sodium in ethanol.

### Intermediates

*7-(1,1-Dimethylheptyl)-5-hydroxy-2,2-dimethylchroman-4-one.* 5-(1,1-Dimethylheptyl) resorcinol (1.0 g, 4.2 mmol) and 3,3-dimethylacrylic acid (0.64 g, 6.3 mmol) both dissolved in methanesulfonic acid (8 mL) were added to P₂O₅ (0.5 g, 3.5 mmol) under nitrogen atmosphere. Then, the reaction mixture was stirred 2 h at 70 °C. Afterwards, water was added, and the product was extracted with EtOAc. The combined organic laylntermedaers were dried over MgSO_{4.} The organic solvent was evaporated under reduced pressure and the crude was purified by column chromatography on flash silica gel (hexane/EtOAc, 5:1), obtaining the desired compound as a pale yellow oil (1.00 g, 75 %); (lit⁶: oil); ¹H NMR (300 MHz, CDCl₃) δ (ppm): 11.53 (s, 1H, OH), 6.45 (d, *J* = 1.6 Hz, 1H, 6-H), 6.37 (d, *J* = 1.6 Hz, 1H, 8-H), 2.71 (s, 2H, 3-H), 1.60-1.49 (m, 2H, 2'-H), 1.47 (s, 6H, OC(CH₃)₂), 1.22 (s, 6H, C(CH₃)₂), 1.23-1.17 (m, 6H, 3'-H, 4'-H, 5'-H), 1.11-0.94 (m, 2H, 6'-H) 0.84 ppm (t, *J* = 6.6 Hz, 3H, 7'-H); ¹³C-NMR (75 MHz, CDCl₃) δ (ppm): 197.4 (4-C), 162.6 (7-C), 161.3 (5-C), 159.5 (8a-C), 106.6 (6-C), 105.7 (8-C), 105.3 (4a-C), 78.9 (2-C), 48.1 (3-C), 44.0 (2'-C), 38.7 (1'-C), 31.7, 29.9, 22.6 (3'-C, 4'-C, 5'-C), 28.4 (C(CH₃)₂), 26.7 (OC(CH₃)₂), 24.6 (6'-C), 14.0 ppm (7'-C); HPLC-MS: [A, 80 → 95 %], *t*_{R}: 4.94 min, (95%); MS (ES⁺, m/z) 319 [M + H]⁺. HRMS calcd for C₂₀H₃₀O₃: 318.2195, found: 318.2198.

*7-(1,1-Dimethylheptyl)-5-hydroxy-3-hydrodymethylene-2,2-dimethylchroman-4-one.* Sodium hydride (377.0 mg, 15.7 mmol) and ethyl formate (2.5 mL, 31.4 mmol) were stirred under nitrogen atmosphere in anhydrous toluene (10 mL). Then, 7-(1,1-dimethylheptyl)-5-hydroxy-2,2-dimethylchroman-4-one (1.0 g, 3.14 mmol) was slowly added to the suspension, and it was stirred at room temperature overnight. Water was added and the product was extracted with EtOAc. The combined organic layers were dried over MgSO₄ and the solvent was evaporated under reduced pressure to obtain a yellow oil (1.07 g, 98 %). The product was used in the next step without further purification. ¹H-RMN (400 MHz, CDCl₃). δ (ppm): 13.41 (d, *J* = 11.6 Hz, 1H, CHOH), 11.20 (s, 1H, 5-OH), 7.28 (d, *J* = 11.6 Hz, 1H, CHOH), 6.38 (d, *J* = 1.6 Hz, 1H, 6-H), 6.30 (d, *J* = 1.6 Hz, 1H, 8-H), 1.49-1.43 (m, 2H, 2'-H), 1.40 (s, 6H, O(C(CH₃)₂)), 1.22-1.18 (m, 2H, 3'-H), 1.15 (s, 6H, 1'-H (C(CH₃)₂)), 1.14-1.09 (m, 4H, 4'-H y 5'-H), 1.02-0.98 (m, 2H, 6'-H), 0.77 (t, *J* = 6.9 Hz , 3H, 7'-H). ¹³C NMR (75 MHz, CDCl₃) δ (ppm): 189.4 (4-C), 162.7 (7-C), 161.6 (5-C), 161.5 (-CHOH), 158.7 (8a-C), 114.4 (3-C), 107.4 (6-C), 106.2 (8-C), 104.9 (4a-C), 78.3 (2-C), 44.4 (2'-C), 38.8 (1'-C), 31.7, 29.9, 22.6 (3'-C, 4'-C, 5'-C), 28.4 (C(*C*H₃)₂), 28.2 (OC(*C*H₃)₂), 24.6 (6'-C), 14.1 ppm (7'-C). HPLC-MS: [A, 80 → 95 %], *t*_{R}: 7.05 min, (99 %); MS (ES⁺, m/z) 347 [M + H]⁺. HRMS calcd for C₂₁H₃₀O₄: 346.2144, found: 346.2146.

*7-(1,1-Dimethylheptyl)-5-hydroxy-3-(pyrrolidine-1-yl-methylene)-2,2-dimethylchroman-4-one.* Pyrrolidine (0.31 mL, 3.70 mmol) was added to a solution of 7-(1,1-dimethylheptyl)-5-hydroxy-3-hydrodymethylene-2,2-dimethylchroman-4-one (1.06 g, 3.08 mmol) in toluene (4 mL). The reaction mixture was stirred under nitrogen atmosphere at room temperature during 5 h. Water was added, and the crude was extracted with EtOAc, washed with brine, dried over anhydrous MgSO₄, filtered and evaporated under reduced pressure. A red crude was purified by medium pressure column chromatography using silica gel with a gradient 0-50 % EtOAc in hexane to obtain a yellow oil (562.2 mg, 46 %). ¹H NMR (400 MHz, CDCl₃) δ (ppm): 13.23 (s, 1H, -OH), 6.86 (s, 1H, -CHN-), 6.40 (d, *J* = 1.7 Hz, 1H, 6-H), 6.28 (d, *J* = 1.7 Hz, 1H, 8-H), 3.39 (br s, 4H, 1 "-H), 1.96 - 1.90 (m, 4H, 2"-H), 1.57 (s, 6H, OC(CH₃)₂), 1.54 - 1.48 (m, 2H, 2'-H), 1.23 - 1.13 (m, 12H, C(CH3)2, 3'-H, 4'-H, 5'-H), 1.09 - 0.99 (m, 2H, 6'-H), 0.82 (t, *J* = 6.9 Hz, 3H, 7'-H). ¹³C NMR (101 MHz, CDCl₃) δ (ppm): 183.3 (CO), 161.5 (5-C), 159.1 (5-C), 157.8 (8a-C), 145.4 (*C*H*-pyrrolidine*)*,* 106.7 (3-C), 106.6 (6-C), 105.8 (4a-C), 104.9 (8-C), 81.2 (2-C), 54.0 (*pyrrolidine*)*,* 44.3 (2'-C), 38.5 (1'-C), 29.01 (C(*C*H₃)₂), 28.7 (-O*C*(CH₃)₂), 25.5 (*pyrrolidine*)*,* 24.7 (6'-C), 31.8, 30.1, 22.75 (3'-C, 4'-C, 5'-C), 14.18 (7'-C). HPLC-MS: [A, 80 → 95 %], *t*_{R}= 5.72 min, (99 %); MS (ES⁺, *m*/*z*) 400 [M+H]⁺. HRMS calcd for C₂₅H₃₇NO₃: 399.2773, found: 399.2789.

**EXAMPLE 1. 8-(1,1-Dimethylheptyl)-5,5-dimethylchromeno[4,3-*d*]pyrimidin-10-ol**. A solution of formamidine (60.45 mg, 0.75 mmol) in EtOH (1.5 mL) was slowly added to a stirred solution of sodium in EtOH (0.5 mL) at room temperature under nitrogen atmosphere. The mixture was stirred for 20 min at the same temperature and the sodium salt was filtered. Then, a solution of 7-(1,1-dimethylheptyl)-5-hydroxy-3-(pyrrolidine-1-yl-methylene)-2,2-dimethylchroman-4-one (150.0 mg, 0.38 mmol) in EtOH (4 mL) was added to the filtered crude and stirred at reflux during 20 h. After cooling, EtOH was evaporated under vacuum. Water was added and the crude was extracted with EtOAc, washed with water and brine, dried over anhydrous MgSO₄, filtered and solvent was evaporated under vacuum. The red oil crude was purified by medium pressure chromatography (Biotage Isolera) on a silica gel column with a gradient of 0 → 100% EtOAc in hexane to obtain example 1 as a yellow oil (99.5 mg, 75 %). ¹H NMR (400 MHz, CDCl₃) δ (ppm): 12.19 (s, 1H, OH), 8.96 (s, 1H, 2-H), 8.47 (s, 1H, 4-H), 6.57 (d, *J* = 1.7 Hz, 1H, 7-H), 6.42 (d, *J* = 1.7 Hz, 1H, 9-H), 1.69 (s, 6H, OC(CH3)2)), 1.57 - 1.51 (m, 2H, 2'-H), 1.24 (s, 6H, -C(CH3)2), 1.21 - 1.15 (m, 6H, 3'-H, 4'-H, 5'-H), 1.10 - 1.01 (m, 2H, 6'-H), 0.82 (t, *J* = 6.8 Hz, 3H, 7'-H). ¹³C NMR (101 MHz, CDCl₃) δ (ppm): 159.6 (2-C), 159.0 (8-C), 155.8, 155.7 (6a-C, 10-C), 155.5 (10b-C), 151.1 (4-C), 129.5 (4a-C), 108.4, 106.3 (7-C, 9-C), 102.0 (10a-C), 77.2 (5-C), 44.3 (2'-C), 38.5 (1'-C), 28.7 (-OC(CH3)2), 27.8 (-C(CH3)2), 31.8, 30.0, 22.7 (3`-C, 4'-C, 5'-C), 24.7 (6'-C), 14.2 (7'-C). HPLC-MS: [A, 80 → 95 %], *t*_{R} = 5.13 min, (99 %); MS (ES⁺, *m*/*z*) 355 [M+H]⁺. HRMS calcd for C₂₂H₃₀N₂O₂: 354.23073, found: 354.23118.

**EXAMPLE 2. 8-(1,1-Dimethylheptyl)-10-methoxy-5,5-dimethylchromeno[4,3-*d*]pyrimidine**. A solution of 7-(1,1-dimethylheptyl)-5-hydroxy-3-(pyrrolidine-1-yl-methylene)-2,2-dimethylchroman-4-one (40.0 mg, 0.1 mmol) in THF (3 mL) was slowly added to a precooled suspension of NaH (4.0 mg, 0.2 mmol) in THF (1.5 mL) under nitrogen atmosphere and the mixture was stirred for 10 min. Then, iodomethane (31 µL, 0.5 mmol) was added and stirred at room temperature overnight. After cooling, solvent was evaporated under vacuum. Water was added to the solution. The product was then extracted with EtOAc, washed with brine, dried over anhydrous MgSO_{4.} Then, the solvent was evaporated under vacuum. The yellow oil crude was purified by medium pressure chromatography (Biotage Isolera) on a silica gel column with 1:1 hexane/EtOAc as eluent to obtain example 2 as a yellow oil (30 mg, 73 %). ¹H NMR (400 MHz, CDCl₃) δ (ppm): 9.12 (s, 1H, 2-H), 8.42 (s, 1H, 4-H), 6.55 (d, *J* = 1.7 Hz, 1H, 7-H), 6.52 (d, *J* = 1.7 Hz, 1H, 9-H), 3.94 (s, 3H, -OCH₃), 1.60 (s, 6H, OC(CH₃)₂), 1.54 - 1.48 (m, 2H, 2'-H), 1.22 (s, 6H, -C(CH₃)₂), 1.18 - 1.08 (m, 6H, 3'-H, 4'-H, 5'-H), 1.04 - 0.94 (m, 2H, 6'-H), 0.77 (t, *J* = 6.8 Hz, 3H, 7'-H). ¹³C NMR (101 MHz, CDCl₃) δ (ppm): 159.1 (2-C), 158.1 (8-C), 157.5, 157.1 (6a-C, 10-C), 155.0 (10b-C), 150.7 (4-C), 130.8 (4a-C), 107.9 (10a-C), 108.9, 103.8 (7-C, 9-C), 76.3 (5-C), 56.6 (-OCH₃), 44.4 (2'-C), 38.6 (1'-C), 28.7 (O*C*(CH₃)₂), 26.6 (-C(*C*H₃)₂), 31.8, 30.0, 22.7 (3'-C, 4'-C, 5'-C), 24.7 (6'-C), 14.2 (7'-C). HPLC-MS: [A, 60 → 95 %], *t*_{R} = 6.35 min, (99 %); MS (ES⁺, *m*/*z*) 369 [M+H]⁺. HRMS calcd for C₂₂H₃₀N₂O₂: 368.24638, found: 368.24605.

**EXAMPLE 3. 10-Benzyloxy-8-(1,1-dimethylheptyl)-5,5-dimethylchromeno[4,3-d]pyrimidine.** A solution of 7-(1,1-dimethylheptyl)-5-hydroxy-3-(pyrrolidine-1-yl-methylene)-2,2-dimethylchroman-4-one (0.15 g, 0.4 mmol) in THF (5 mL) was slowly added to a precooled suspension of sodium hydride (15.2 mg, 0.6 mmol) in THF (2 mL) and the mixture was stirred for 10 min under nitrogen atmosphere. Then, benzyl bromide (151 µL, 1.3 mmol) was added and stirred at reflux overnight. After cooling, solvent was evaporated under vacuum. Water was added and the crude was extracted with EtOAc, washed with brine, dried over anhydrous MgSO4, filtered and the solvent was evaporated under vacuum. The yellow oil crude was purified by medium pressure column chromatography (Biotage Isolera) on silica gel with a gradient of 0 - 50% EtOAc in hexane to obtain example 3 as a yellow oil (95.4 mg, 51 %). ¹H NMR (400 MHz, CDCl₃) δ (ppm): 9.13 (s, 1H, 2-H), 8.41 (s, 1H, 4-H), 7.60 - 7.51 (m, 2H, 2-H_{Bz}), 7.32 - 7.26 (m, 2H, 3-H_{Bz}), 7.23 - 7.17 (m, 1H, 4-H_{Bz}), 6.58 (d, *J* = 1.7 Hz, 1H, 7-H), 6.52 (d, *J* = 1.7 Hz, 1H, 9-H), 5.21 (s, 2H, -OCH₂-), 1.59 (s, 6H, - OC(CH₃)₂), 1.48 - 1.41 (m, 2H, 2'-H), 1.17 (s, 6H, -C(CH₃)₂), 1.16 - 1.02 (m, 6H, 3'-H, 4'-H, 5'-H), 0.99 - 0.86 (m, 2H, 6'-H), 0.75 (t, *J* = 7.0 Hz, 3H, 7'-H). ¹³C NMR (101 MHz, CDCl₃) δ (ppm): 158.0 (2-C), 157.9 (8-C), 157.4, 156.8 (6a-C, 10-C), 154.9 (10b-C), 150.6 (4-C), 137.3 (1-C_{Bz}), 130.7 (4a-C), 128.5, 127.1 (2-C_{Bz}, 3-C_{Bz}), 127.7 (4-C_{Bz}), 109.1, 106.7 (7-C, 9-C), 108.7 (10a-C), 76.3 (5-C), 71.5(-OCH₂-), 44.4 (2'-C), 38.5 (1'-C), 28.6 (OC(*C*H₃), 26.6 (-C(*C*H₃)₂), 31.8, 29.9, 22.7 (3'-C, 4'-C, 5'-C), 24.6 (6'-C), 14.1 (7'-C). HPLC-MS: [A, 80 → 95 %], *t*_{R} = 5.12 min, (99 %); MS (ES⁺, *m*/*z*) 445 [M+H]⁺. HRMS calcd for C₂₂H₃₀N₂O₂: 444.2777, found: 444.2796.

### EXAMPLE 4. Biological assays

In the present invention, cannabinoid affinity was evaluated using Homogeneous Time Resolved Fluorescence (HTFR) binding assays. Cannabinoid activity was assessed using an array of functional assays including cAMP, MAPK and barrestin2 recruitment in CB1 and CB2 transfected HEK293 cells. Moreover, cannabinoid functionality was proved in primary neuronal cultures. Primary neuronal cultures are an important model of the nervous system used for drug discovery of neurodegenerative diseases and neuroinflammation disorders. The predictive capacity of this culture assay allows the identification of novel therapeutic agents for these diseases and disorders.

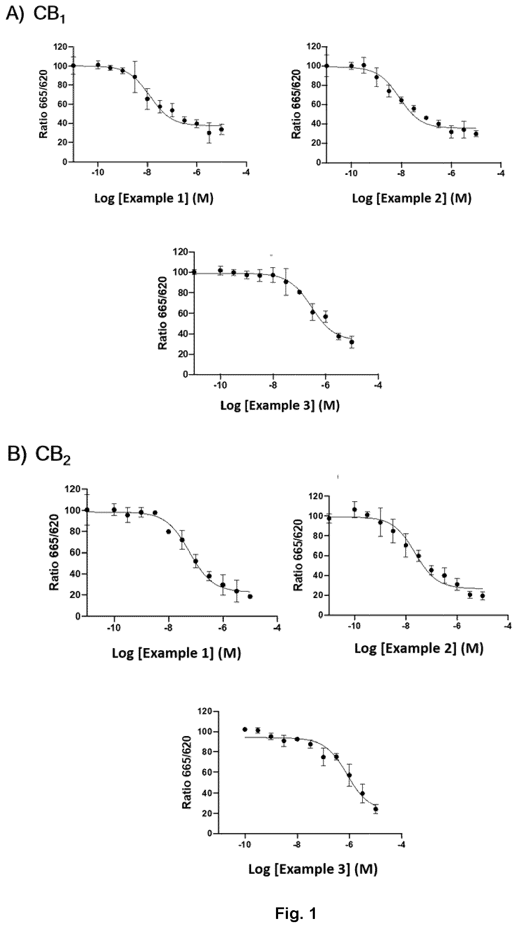

Figure 1 demonstrates that the claimed compounds exhibit affinity for both cannabinoid receptors CB1 and CB2 in (HTRF) binding assays.

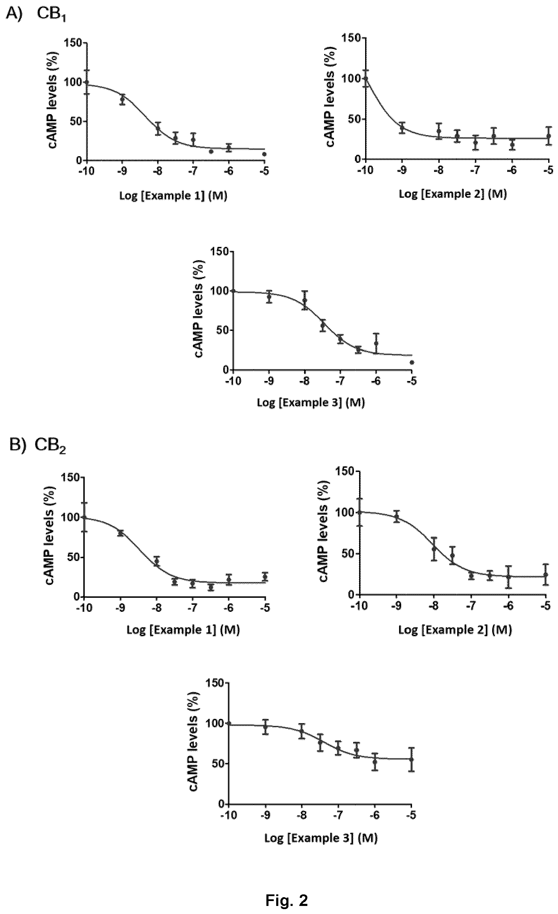

Figure 2 demonstrates that Example 1, Example 2 and Example 3 behave as CB1 and CB2 potent and efficacious agonists in cAMP assays.

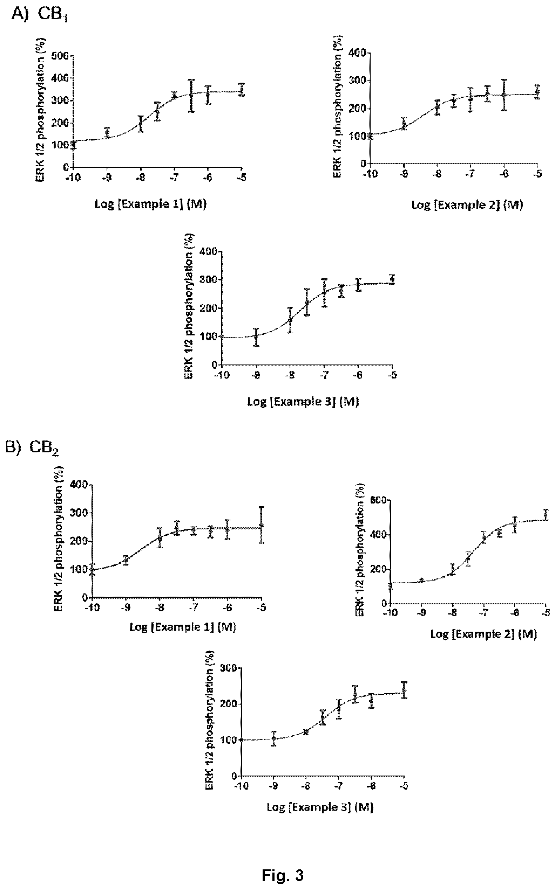

Figure 3 demonstrates that compounds Example 1, Example 2 and Example 3 act as agonists in MAPK assays in HEK293-CB1 and HEK293-CB2 cells.

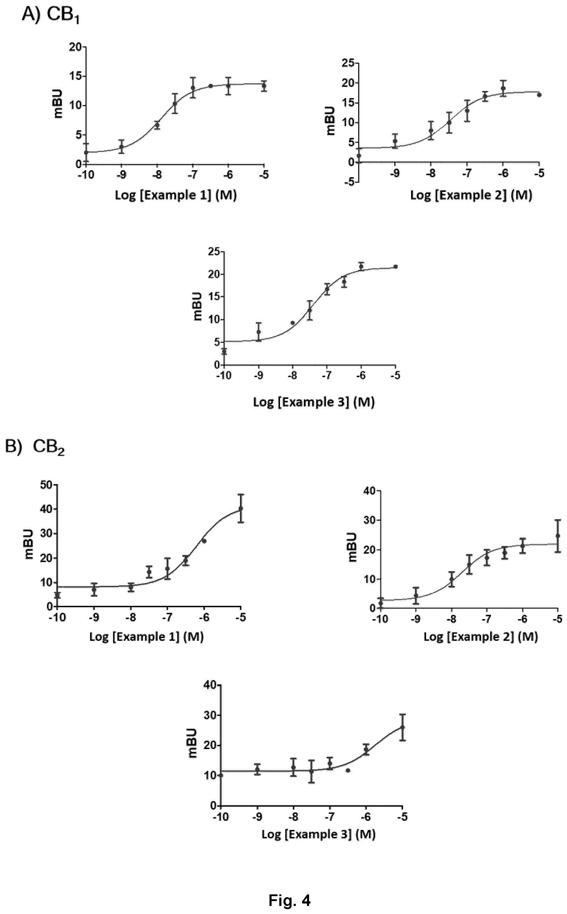

Figure 4 demonstrates that Example 1, Example 2 and Example 3 recruit β-arrestin2 in HEK293-CB1 and HEK293-CB2 cells.

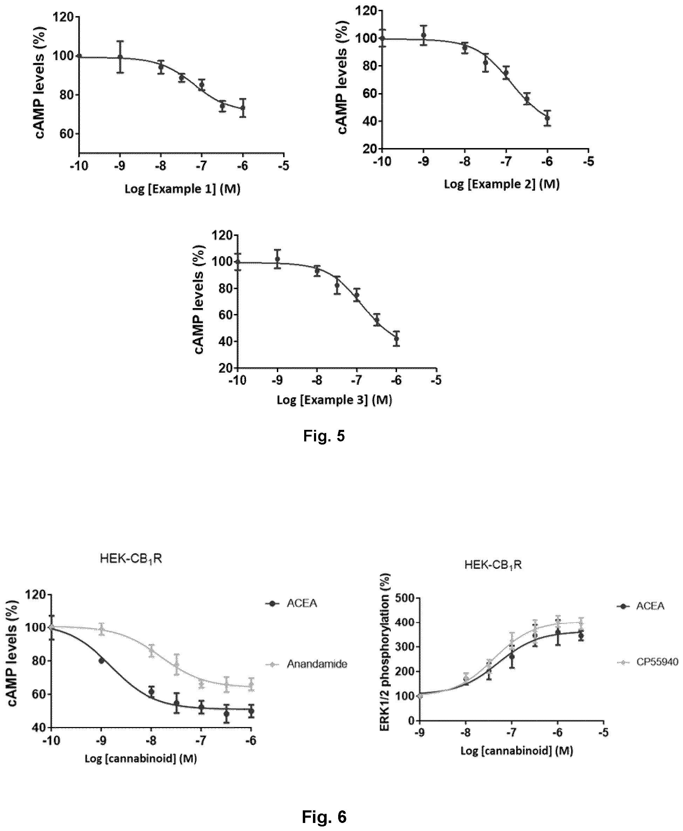

Figure 5 demonstrate cannabinoid agonistic activity of Example 1, Example 2 and Example 3 in primary neuronal cultures using cAMP assays.

Figure 6 shows the activity of reference cannabinoids ACEA and anandamide in cAMP and MAPK assays in HEK293-CB1 cells.

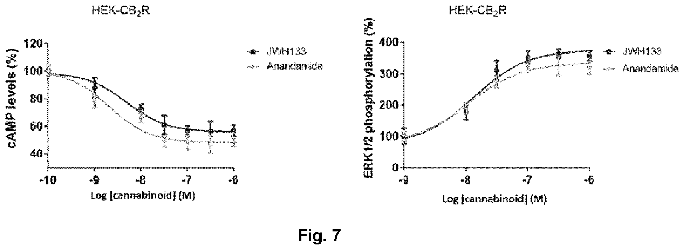

Figure 7 shows the activity of reference cannabinoids JWH133 and anandamide in cAMP and MAPK assays in HEK293-CB2 cells.

### Methodology

### HTRF competitive binding assays

SNAP CB2R were expressed in HEK-293T cells. For SNAP protein labeling, cell culture medium was removed from the 25-cm2 flask and 100 nM SNAP-Lumi4-Tb, previously diluted in 3 mL of TLB 1X, was added to the flask and incubated for 1 h at 37 °C under 5% CO₂ atmosphere in a cell incubator. Cells were then washed four times with 2 mL of TLB 1X to remove the excess of SNAP-Lumi4-Tb, detached with enzyme-free cell dissociation buffer, centrifuged 5 min at 1500 rpm and collected in 1 mL of TLB 1X. Tag-lite-based binding assays were performed 24 h after transfection. Densities of 2,500-3,000 cells/well were used to perform binding assays in white opaque 384-well plates. For competition binding assays, the fluorophore-conjugated CB2R ligand (labeled CM157), and compounds, Example compounds of the present invention, were diluted in TLB 1X. HEK-293T cells transiently expressing Tb labeled SNAP-CB2R with or without CB1R were incubated with 20 nM fluorophore-conjugated CB2R ligand, in the presence of increasing concentrations (0-10 µM range) of Example compounds. Plates were then incubated for at least 2 h at room temperature before signal detection. Homogeneous time-resolved fluorescence energy transfer (HTRF) was detected using a PHERAstar Flagship microplate reader (Perkin-Elmer, Waltham, MA, USA) equipped with a FRET optic module allowing donor excitation at 337 nm and signal collection at both 665 and 620 nm.

### cAMP assays

Two hours before initiating the experiment, HEK-293T cells medium was replaced by serum-starved DMEM medium. Then, cells were detached and resuspended in DMEM medium containing 50 µM zardaverine. Then, cells were plated in 384-well microplates (2500 cells/well), pretreated (15 min) with the vehicle and stimulated with the Example compounds of the patent or reference cannabinoids (anadamide, ACEA, JWH-133) or vehicule (15 min) before adding 0.5 µM forskolin or vehicle. Readings were performed after 60 min incubation at 25°. Homogeneous time-resolved fluorescence energy transfer (HTRF) measures were performed using the Lance Ultra cAMP kit (PerkinElmer, Waltham, MA, USA). Fluorescence at 665 nm was analysed on a PHERAstar Flagship microplate reader equipped with an HTRF optical module (BMG Lab technologies, Offenburg, Germany).

### β-Arrestin 2 recruitment assays

β-Arrestin recruitment was determined with BRET experiments performed in HEK-293T cells 48 h after transfection with the cDNA for either CB1R-YFP, CB2R-YFP or CB2R-YFP and CB1R, and 1 µg cDNA corresponding to β-arrestin 2-RLuc. Cells (20 µg protein) were distributed in 96-well microplates (Corning 3600, white plates with white bottom) and incubated with compounds, Example compounds of the patent, for 10 min prior to the addition of 5 µM coelenterazine H (Molecular Probes, Eugene, OR). 1 min after coelenterazine H addition, BRET readings corresponding to β-arrestin 2-Rluc and receptor-YFP were quantified. The readings were collected using a Mithras LB 940 (Berthold Technologies, Bad Wildbad, Germany) that allows the integration of the signals detected in the short-wavelength filter at 485 nm and the longwavelength filter at 530 nm. To quantify protein-RLuc expression, luminescence readings were performed 10 min after addition of 5 µM coelenterazine H.

### MAPK assays

To determine ERK1/2 phosphorylation, 40,000 cells/well were plated in transparent Deltalab 96-well microplates and kept at the incubator for 24 h. 2-4 h before the experiment, the medium was substituted by serum-starved DMEM medium. Then, cells were stimulated at 25 °C for 7 min with compounds, Example compound of the patent, or reference cannabinoids (anadamide, ACEA, JWH-133) or vehicle in serum-starved DMEM medium. Cells were then washed twice with cold PBS before addition of lysis buffer (20 min treatment). 10 µL of each supernatant were placed in white ProxiPlate 384-well microplates and ERK1/2 phosphorylation was determined using AlphaScreen^{®}SureFire^{®} kit (Perkin Elmer) following the instructions of the supplier and using an EnSpire^{®} Multimode Plate Reader (PerkinElmer, Waltham, MA, USA).

### Primary Neuronal culture

Primary cultures of cortical neurons were obtained from fetal Sprague Dawley rats (embryonic day 20). Cells were isolated as described in Hradsky et al. (2013) and plated at a confluence of 40,000 cells/0.32 cm₂ in 96 well plates for MAPK experiments and in 6 well plates for cAMP assays. Cells were maintained in Neurobasal medium supplemented with 2mM L-glutamine, 100 U/ml penicillin/streptomycin, and 2% (v/v) B27 supplement (GIBCO) for 12 days.

## Claims

1. A compound of formula (I) or a pharmaceutically acceptable salt thereof: 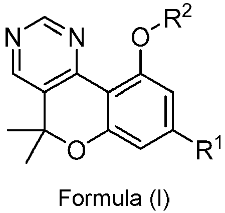 wherein:
R¹ is selected from substituted or unsubstituted C₁-C₉ alkyl;
R² is selected from hydrogen, substituted or unsubstituted C₁-C₆ alkyl, substituted or unsubstituted C₅-C₂₀ aryl, substituted or unsubstituted C₃-C₉ cycloalkyl, or substituted or unsubstituted heterocyclic ring,
for use as a medicament.

2. Compound for use, according to claim 1, wherein R¹ is selected from methyl, pentyl, 1,1-cyclopentylheptyl, and 1,1-dimethylheptyl.

3. Compound for use, according to claim 1 or 2, wherein R² is a substituted or unsubstituted C₁-C₄ alkyl group.

4. Compound for use, according to claim 3, wherein R² is a C₁-C₄ alkyl group selected from methyl, ethyl, iso-propyl, and tert-butyl.

5. Compound for use, according to claim 3, wherein R² is a C₁-C₄ alkyl group substituted by an aryl group.

6. Compound for use, according to claim 5, wherein R² is benzyl.

7. Compound for use, according to claim 1 or 2, wherein R² is a substituted or unsubstituted C₅-C₁₂ aryl group.

8. Compound for use, according to claim 7, wherein R² is selected from phenyl and phenyl substituted by a hydroxyl or a methoxy group.

9. Compound for use, according to claim 1 or 2, wherein R² is a substituted or unsubstituted C₅-C₇ cycloalkyl group.

10. Compound for use, according to claim 9, wherein R² is cyclohexyl.

11. Compound for use, according to claim 1 or 2, wherein R² is H.

12. Compound for use, according to claim 1, wherein the compound is selected from:
8-(1,1-dimethylheptyl)-5,5-dimethylchromeno[4,3-d]pyrimidin-10-ol,
8-(1,1-dimethylheptyl)-10-methoxy-5,5-dimethylchromeno[4,3-d]pyrimidine, and
10-benzyloxy-8-(1,1-dimethylheptyl)-5,5-dimethylchromeno[4,3-d]pyrimidine.

13. Compound of formula (I), as defined in any of claims 1 to 12, or a pharmaceutically acceptable salt thereof, for use in the prevention and/or the treatment of inflammatory disorders associated with the cannabinoid receptors selected from neuropathic pain, and neurodegenerative diseases selected from Alzheimer's, Parkinson's, and Huntington's diseases.

14. A compound of formula (I) or a pharmaceutically acceptable salt thereof: 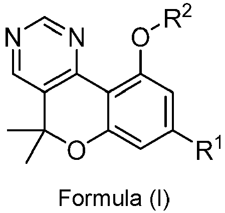
wherein R¹ and R² are as defined in any of claims 1 to 12,
and provided that the compound 5,5-dimethyl-8-pentyl-5H-chromeno[4,3-d]pyrimidin-10-ol is excluded from the formula (I).

15. A Pharmaceutical composition comprising a compound of formula (I), as defined in claim 14, or a pharmaceutically acceptable salt thereof and at least one excipient, adjuvant and/or pharmaceutically acceptable carrier.
